# EUROPEAN PATENT APPLICATION

(11) **EP 1 394 242 A2**
(43) Date of publication of application: **03.03.2004**
(21) Application number: 03254692.1
(22) Date of filing: 28.07.2003
(51) Int. Cl.: C10M 135/16, C07D 235/02, C07D 239/78, C07D 243/22

(54) **Cyclic thioureas and their use as additives for lubricating oils**

(30) Priority: 07.08.2002 US 401539 P
(71) Applicant: ROHM AND HAAS COMPANY, Philadelphia, Pennsylvania 19106-2399 (US)
(72) Inventor: Mukkamala, Ravindranath, Lansdale, Pennsylvania 19446 (US)
(74) Representative: Kent, Venetia Katherine

(57) **Abstract**

A composition comprising: (a) from 0.1% to 20% of at least one compound comprising: (i) a five-, six-, or seven-membered ring containing a thiourea functional group; and (ii) at least one of: (A) a carbocyclic ring system fused to the five-, six-, or seven-membered ring; and (B) an aryl, aralkyl, C₄-C₂₂ alkyl or C₄-C₂₂ alkenyl group attached to a ring carbon atom of the five-, six-, or seven-membered ring; and (b) a lubricating oil.

## Description

### Background

This invention relates generally to bicyclic thioureas useful as additives for lubricating oils.

Zinc dialkyldithiophosphates (ZDDP) are widely used as lubricant additives. The principal disadvantages of these compounds are that an ash residue is produced by the zinc as the additive is consumed, and that phosphorus is known to affect the efficiency of catalytic converters in motor vehicles, thereby causing emissions problems. Cyclic thiourea compounds useful as lubricant additives are disclosed in U.S. Patent No. 5,935,913. However, the compounds disclosed therein are not within the scope of the present invention.

The problem addressed by this invention is to find additional non-metallic, non-phosphorus-containing oil-soluble additives for lubricating oils.

### Statement of Invention

The present invention is directed to a composition comprising: (a) from 0.1% to 20% of at least one compound comprising: (i) a five-, six-, or seven-membered ring containing a thiourea functional group; and (ii) at least one of:
(A) a carbocyclic ring system fused to the five-, six-, or seven-membered ring; and
(B) an aryl, aralkyl, C₄-C₂₂ alkyl or C₄-C₂₂ alkenyl group attached to a ring carbon atom of the five-, six-, or seven-membered ring; and (b) a lubricating oil.

The present invention is further directed to a method for improving the anti-wear characteristics of a lubricating oil by adding from 0.1% to 20% of a compound having a five-, six-, or seven-membered ring containing a thiourea functional group and at least one of: (i) a carbocyclic ring system fused to the five-, six-, or seven-membered ring. and (ii) an aryl, aralkyl, C₄-C₂₂ alkyl or C₄-C₂₂ alkenyl group attached to a ring carbon atom of the five-, six-, or seven-membered ring.

The present invention is further directed to novel compounds having a five-, six-, or seven-membered ring containing a thiourea functional group and a carbocyclic ring system fused to the five-, six-, or seven-membered ring.

### Detailed Description

All percentages are weight percentages based on the entire composition described, unless specified otherwise. An "alkyl" group is a saturated hydrocarbyl group having from one to twenty-two carbon atoms in a linear, branched or cyclic arrangement, and having from 0 to 2 oxygen, nitrogen or sulfur atoms. Substitution on alkyl groups of one or more halo, hydroxy, alkoxy, alkanoyl or amido groups is permitted, alkoxy, alkanoyl and amido groups may in turn be substituted by one or more halo substituents. In one preferred embodiment, alkyl. groups contain from one to twelve carbon atoms and from 0 to 1 oxygen, nitrogen or sulfur atoms; in another preferred embodiment, alkyl groups contain from 4 to 22 carbon atoms, and more preferably, no heteroatoms. An "alkenyl" group is an "alkyl" group in which at least one single bond has been replaced with a double bond. A "difunctional alkyl" or "difunctional alkenyl" group is an alkyl or alkenyl group having two points of attachment on the same or different carbon atoms, e.g., -CH₂-, -CH₂CH₂-, -CH(CH₂CH₃)-, and -CH=CH-. An "aryl" group is a substituent derived from an aromatic compound, including heterocyclic aromatic compounds having heteroatoms chosen from among nitrogen, oxygen and sulfur. An aryl group has a total of from five to twenty ring atoms, and has one or more rings which are separate or fused. Substitution on aryl groups of one or more halo, alkyl, alkenyl, hydroxy, alkoxy, alkanoyl or amido groups is permitted, with substitution by one or more halo groups being possible on alkyl, alkenyl, alkoxy, alkanoyl or amido groups. An "aralkyl" group is an "alkyl" group substituted by an "aryl" group. Rings which are fused have ring atoms in common, e.g., in any pair of fused rings, the two rings have at least two ring atoms in common. A "carbocyclic ring system" is one or more cycloalkyl, cycloalkenyl or aryl rings with a total of 5 to 22 ring atoms, all of which are carbon atoms. When a carbocyclic ring system has more than one ring, preferably the rings are fused to each other, i.e., each pair of rings has at least two carbon atoms in common. A "lubricating oil" is a natural or synthetic oil, or a mixture thereof, having suitable viscosity for use as a lubricant, e.g., as crankcase oil in an internal combustion engine, automatic transmission fluid, turbine lubricant, gear lubricant, compressor lubricant, metal-working lubricant, hydraulic fluid, etc.

In a preferred embodiment, compounds used in this invention have: (i) a five- or six-membered ring containing a thiourea functional group; and (ii) at least one of: (A) a carbocyclic ring system fused to the five-, six-, or seven-membered ring; and (B) a C₈-C₂₂ alkyl or C₈-C₂₂ alkenyl group attached to a ring carbon atom of the five- or six-membered ring.

Compounds of this invention are prepared, for example, by condensation of carbon disulfide with a diamino compound, with elimination of hydrogen sulfide to form a cyclic thiourea. When the diamino compound is carbocyclic, the product has a cyclic thiourea fused to a carbocyclic ring system. Acyclic diamines give monocyclic thioureas.

In a preferred embodiment of this invention, the compound has formula (I) 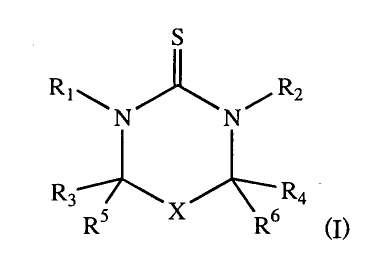 wherein X represents a single bond, CR⁷R⁸ or CR⁷R⁸CR⁹R¹⁰; R¹ and R² independently are hydrogen, alkyl, alkenyl, aryl, aralkyl, alkanoyl, aroyl, - CHR¹¹CHR¹²COOR¹³, -CR¹⁴R¹⁵-NHR¹⁶ or -C(Z)NHR¹⁷; Z is S or O; and R³, R4, R⁵, R⁶, R⁷, R⁸, R⁹ and R¹⁰ independently are hydrogen, alkyl, alkenyl, aryl, aralkyl or at least two of R¹, R², R³, R⁴, R⁵, R⁶, R⁷, R⁸, R⁹ and R¹⁰ combine to form a carbocyclic ring system fused to the five-, six- or seven-membered ring; provided that: (i) at least one carbocyclic ring system is fused to the five-, six- or seven-membered ring; or (ii) at least one of R³, R⁴, R⁵, R⁶, R⁷, R⁸, R⁹ and R¹⁰ is an aryl, aralkyl, C₄-C₂₂ alkyl or C₄-C₂₂ alkenyl group.

In one embodiment, at least one carbocyclic ring system is fused to the five-, six- or seven-membered ring. In another embodiment, at least one of R³, R⁴, R⁵, R⁶, R⁷, R⁸, R⁹ and R¹⁰ is a C₈-C₂₂ alkyl or C₈-C₂₂ alkenyl group. In another embodiment, at least one of R¹ and R² is -CHR¹¹CHR¹²COOR¹³, -CR¹⁴R¹⁵-NHR¹⁶ or -C(Z)NHR¹⁷. In another embodiment of the invention at least one of R¹, R², R³, R⁴, R⁵, R⁶, R⁷, R⁸, R⁹ and R¹⁰ has at least six carbon atoms; more preferably at least one of R¹, R², R³, R⁴, R⁵, R⁶, R⁷, R⁸, R⁹ and R¹⁰ has at least eight carbon atoms; and most preferably at least one of R¹, R², R³, R⁴, R⁵, R⁶, R⁷, R⁸, R⁹ and R¹⁰ has at least ten carbon atoms.

In one preferred embodiment of the invention, X is a single bond, and R⁵ and R⁶ combine to form a five- or six-membered carbocyclic ring fused to the 4-and 5-positions of an imidazolidine-2-thione ring. Preferably, the five- or six-membered carbocyclic ring is a cycloalkyl ring, as shown in formula (II) 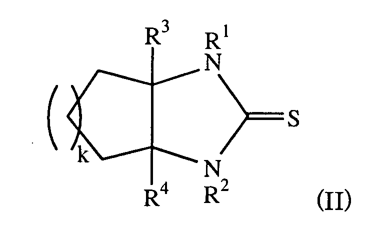 wherein k is one or two. Preferably, R³ and R⁴ do not combine to form an additional carbocyclic ring system. In this embodiment, the five-membered ring containing the thiourea group and the five- or six-membered carbocyclic ring share two carbon atoms. Compounds of formula (II) are prepared, for example, from reaction of carbon disulfide with 1,2-cyclic diamines, as shown for the cyclohexyl (k=2) compound in Scheme 1.

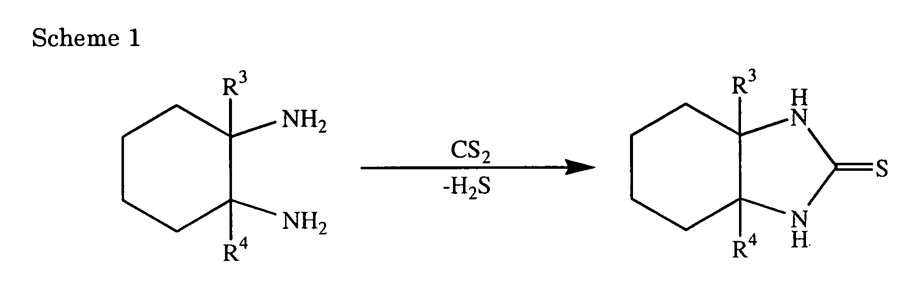

In another preferred embodiment, R² and R⁶ combine to form a five- or six-membered cycloalkyl ring fused to one thiourea nitrogen and to the adjacent ring carbon. When X is a single bond, and R³, R⁴ and R⁵ are hydrogen, the resulting compound has formula (III)

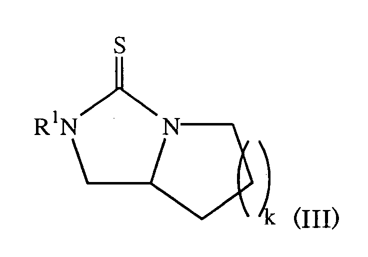

In another preferred embodiment, X is CR⁷R⁸ and the six-membered ring containing the thiourea group is fused to a substituted polycyclic aromatic hydrocarbon nucleus, i.e., R³, R⁴, R⁵, R⁶, R⁷ and R⁸ in formula (I) combine to form a substituted polycyclic aromatic ring system. Preferably, the polycyclic aromatic hydrocarbon nucleus is substituted by alkyl or alkenyl groups to improve the oil solubility of the compound, most preferably the polycyclic aromatic hydrocarbon nucleus is substituted by at least one alkyl or alkenyl group having at least eight carbon atoms. For example, in one embodiment, a six-membered ring containing a thiourea functional group, and fused to a substituted naphthalene would have the structure shown in formula (IV) 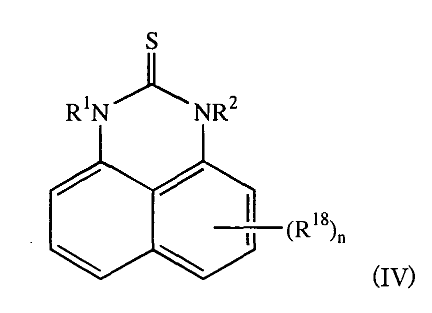 wherein R¹⁸ represents alkyl or alkenyl groups which may be the same or different, and n is an integer from zero to six. Preferably, at least one of R¹, R² and the R¹⁸ group(s) contains at least six carbon atoms, more preferably at least eight carbon atoms, and most preferably at least ten carbon atoms. Preferably, at least one of the R¹⁸ groups contains at least six carbon atoms, more preferably at least eight carbon atoms, and most preferably at least ten carbon atoms. Preferably, all of the R¹⁸ groups contain at least four carbon atoms, more preferably at least six carbon atoms. Compounds of formula (IV) can be prepared, for example, from reaction of a substituted 1,8-diaminonaphthalene with carbon disulfide.

In another preferred embodiment, X is CR⁷R⁸CR⁹R¹⁰, and R³ and R⁴ combine to form a -CH₂- or -CH₂CH₂- group bridging the seven-membered thiourea ring, resulting in the structure depicted in formula (V) 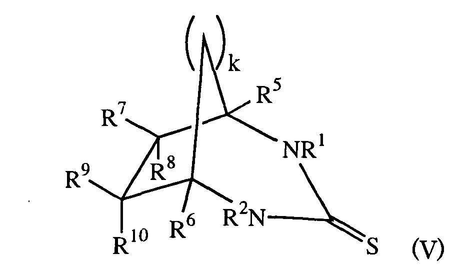 In this embodiment, the thiourea-containing ring and the five- or six-membered carbocyclic ring share four carbon atoms, i.e., the ones to which R⁵, R⁷, R⁹ and R⁶ are attached. Compounds of formula (V) can be prepared, for example, from the reaction of cis-1,4-diaminocyclohexanes (k=2) or cis-1,3-diaminocyclopentanes (k=1) with carbon disulfide.

In one aspect of the invention, a compound in which one or both of the thiourea nitrogen atoms bear a hydrogen atom is allowed to react with a compound of formula CHR¹¹=CR¹²COOR¹³ to produce a compound having a - CHR¹¹CHR¹²COOR¹³ group on a thiourea nitrogen atom. Preferably, R¹¹ and R¹² are hydrogen or methyl. Preferably, the compound of formula CHR¹¹=CR¹²COOR¹³ is an alkyl or aralkyl acrylate having R¹¹= R¹²=H and R¹³=alkyl or aralkyl; or a methacrylate ester having R¹¹=H and R¹²=CH₃; or a crotonate ester having R¹²=H and R¹¹=CH₃. In another aspect of this invention, a compound in which one or both of the thiourea nitrogen atoms bear a hydrogen atom is alkylated with an imine, CR¹⁴R¹⁵=NR¹⁶. Preferably, R¹⁶ is alkyl, most preferably C₁₂-C₂₂ alkyl. In one embodiment, R¹⁶ is derived from an unsubstituted C₁₆-C₂₂ alkyl amine, R¹⁶NH₂, preferably one which is an oil-soluble amine. In one embodiment, the alkyl amine is a tertiary alkyl primary amine, i.e., a primary amine in which the alkyl group is attached to the amino group through a tertiary carbon. Examples of commercially available tertiary alkyl primary amines are the Primene™ amines available from Rohm and Haas Company, Philadelphia, PA. Preferably, R¹⁴ and R¹⁵ independently are alkyl or hydrogen. In a preferred embodiment of the invention, CR¹⁴R¹⁵=NR¹⁶ is a formaldehyde imine, CH₂=NR¹⁶. In another aspect of this invention, a compound in which one or both of the thiourea nitrogen atoms bear a hydrogen atom reacts with an isocyanate, R¹⁷NCO, or an isothiocyanate, R¹⁷NCS, to produce a compound having a -C(O)NHR¹⁷ or a -C(S)NHR¹⁷ group, respectively. Preferably, R¹⁷ is aryl, alkyl or aralkyl, more preferably C₈-C₂₀ alkyl, aryl or aralkyl. A compound in which one or both of the thiourea nitrogen atoms bear a hydrogen atom also may react with an alkyl, alkenyl, aralkyl, alkanoyl or aroyl group bearing a suitable leaving group, thereby introducing an alkyl, alkenyl or aralkyl group on a thiourea nitrogen atom. Suitable leaving groups and conditions for such a reaction are well known to those skilled in the art; suitable leaving groups include, e.g., iodide, bromide, chloride; and ester-derived leaving groups, e.g., alkanoate, mesylate, tosylate and triflate.

Substitution on more than one thiourea nitrogen is possible in the compounds of this invention, either from the same thiourea group or different thiourea groups, also is possible, depending on the stoichiometry and conditions of the reaction.

The composition of the present invention includes at least one compound having a five-, six-, or seven-membered ring containing a thiourea functional group, and at least one of: (i) a carbocyclic ring system fused to the five-, six-, or seven-membered ring; and (ii) an aryl, aralkyl, C₄-C₂₂ alkyl or C₄-C₂₂ alkenyl group attached to a ring carbon atom of the five-, six-, or seven-membered ring. In one embodiment of the invention, the composition contains at least two such compounds, and more preferably contains at least three such compounds. Preferably, the compound(s) is present in a lubricating oil in a total amount of at least 0.2%, more preferably at least 0.3%, and most preferably at least 0.4%. Preferably, the compound(s) is present in a lubricating oil in a total amount no greater than 10%, more preferably no greater than 5%, and most preferably no greater than 2%. Preferably, the compounds are soluble at the aforementioned levels.

Optionally, other additives typically used in lubricating oils are present in the composition. Such additives include, but are not limited to, other anti-wear additives, anti-corrosion additives, dispersants, detergents, antioxidants, antifoamants, friction modifiers, seal swell agents, demulsifiers, viscosity index improvers and pour point depressants. Other anti-wear additives that can be used in combination with the compound having a five- or six-membered ring containing a thiourea functional group and a carbocyclic ring system fused to the five- or six-membered ring include the commercial products known as ZDDP, which are zinc dialkyldithiophosphates. In addition to improving the anti-wear characteristics of lubricating oils, the compounds described herein typically also improve anti-corrosion characteristics and function as anti-oxidants.

## Claims

1. A composition comprising:
(a) from 0.1% to 20% of at least one compound comprising:
(i) a five-, six-, or seven-membered ring containing a thiourea functional group; and
(ii) at least one of :
(A) a carbocyclic ring system fused to the five-, six-, or seven-membered ring; and
(B) an aryl, aralkyl, C₄-C₂₂ alkyl or C₄-C₂₂ alkenyl group attached to a ring carbon atom of the five-, six-, or seven-membered ring; and
(b) a lubricating oil.

2. The composition of claim 1 in which said at least one compound has formula (I) 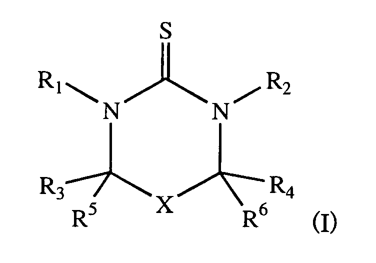 wherein X represents a single bond, CR⁷R⁸ or CR⁷R⁸CR⁹R¹⁰; R¹ and R² independently are hydrogen, alkyl, alkenyl, aryl, aralkyl, alkanoyl, aroyl, - CHR¹¹CHR¹²COOR¹³, -CR¹⁴R¹⁵-NHR¹⁶ or -C(Z)NHR¹⁷; Z is S or O; and R³, R⁴, R⁵, R⁶, R⁷, R⁸, R⁹ and R¹⁰ independently are hydrogen, alkyl, alkenyl, aryl, aralkyl or at least two of R¹, R², R³, R⁴, R⁵, R⁶, R⁷, R⁸, R⁹ and R¹⁰ combine to form a carbocyclic ring system fused to the five-, six- or seven-membered ring;
provided that: (i) at least one carbocyclic ring system is fused to the five-, six- or seven-membered ring; or (ii) at least one of R³, R⁴, R⁵, R⁶, R⁷, R⁸, R⁹ and R¹⁰ is an aryl, aralkyl, C₄-C₂₂ alkyl or C₄-C₂₂ alkenyl group.

3. The composition. of claim 2 in which X represents a single bond, and said at least one compound has formula (II) 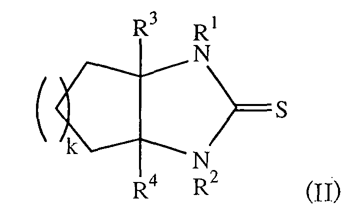 wherein k is one or two.

4. The composition of claim 2 in which X represents a single bond, and said at least one compound has formula (III) 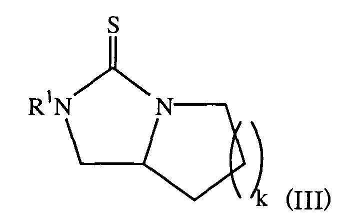 wherein k is one or two.

5. The composition of claim 2 in which X is CR⁷R⁸, and said at least one compound is represented by formula (IV) 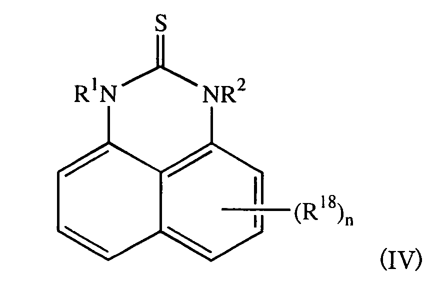 wherein R¹⁸ represents alkyl or alkenyl groups which may be the same or different, and n is an integer from zero to six.

6. The composition of claim 2 in which at least one of R¹ and R² is - CHR¹¹CHR¹²COOR¹³, -CR¹⁴R¹⁵-NHR¹⁶ or -C(Z)NHR¹⁷.

7. A compound having a five-, six-, or seven-membered ring containing a thiourea functional group, and represented by formula I: 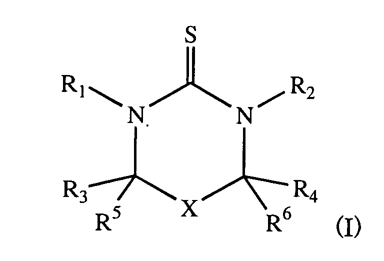 wherein X represents a single bond, CR⁷R⁸ or CR⁷R⁸CR⁹R¹⁰; R¹ and R² independently are hydrogen, alkyl, alkenyl, aryl, aralkyl, alkanoyl, aroyl, - CHR¹¹CHR¹²COOR¹³, -CR¹⁴R¹⁵-NHR¹⁶ or -C(Z)NHR¹⁷; Z is S or O; and R³, R⁴, R⁵, R⁶, R⁷, R⁸, R⁹ and R¹⁰ independently are hydrogen, alkyl, alkenyl, aryl, aralkyl or at least two of R¹, R², R³, R⁴, R⁵, R⁶, R⁷, R⁸, R⁹ and R¹⁰ combine to form a carbocyclic ring system fused to the five-, six- or seven-membered ring;
provided that at least one carbocyclic ring system is fused to the five-, six- or seven-membered ring.

8. The compound of claim 7 in which X represents a single bond

9. The compound of claim 8 in which said at least one compound has formula (II) 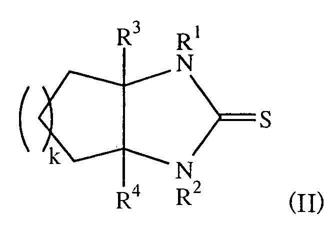 wherein k is one or two.

10. The compound of claim 7 in which at least one of R¹ and R² is - CHR¹¹CHR¹²COOR¹³, -CR¹⁴R¹⁵-NHR¹⁶ or -C(Z)NHR¹⁷.
